# EUROPEAN PATENT APPLICATION

(11) **EP 4 563 690 A1**
(43) Date of publication of application: **04.06.2025**
(21) Application number: 23213134.2
(22) Date of filing: 29.11.2023
(51) Int. Cl.: C12M 1/12, C12M 1/00, C12M 1/42

(54) **BIOREACTOR, GAS-EXCHANGE UNIT, AND SYSTEM FROM THE BIOREACTOR AND THE GAS-EXCHANGE UNIT FOR TISSUE CULTURE OR CELL CULTURE AND RELATED CULTURING METHOD FOR CULTURING A SAMPLE COMPRISING TISSUE OR EMBEDDED CELLS**

(71) Applicant: Eberhard Karls Universität Tübingen Medizinische Fakultät, 72074 Tübingen (DE)
(72) Inventor: SCHÜRCH, Christian, 72070 Tübingen (DE); WEIGELIN, Bettina, 72076 Tübingen (DE); MÄHLEN, Marius, 72076 Tübingen (DE); ZIMMERMANN, Max, 72076 Tübingen (DE); ROTTMANN, Isabelle, 72076 Tübingen (DE)
(74) Representative: Witte, Weller & Partner Patentanwälte mbB

(57) **Abstract**

The present invention relates to a bioreactor for culturing allowing simultaneous live cell imaging of a sample, a gas-exchange unit for allowing gas-exchange of a culture medium during cell culture or tissue culture allowing to keep a bioreactor suitable for long-term storage outside an incubator cabinet, a system from the bioreactor and the gas-exchange unit, and a method for culturing a sample.

## Description

The present invention relates to a bioreactor for culturing and allowing simultaneous live cell imaging of a sample, a gas-exchange unit for allowing gas-exchange of a culture medium during cell culture or tissue culture and allowing to keep a bioreactor suitable for long-term storage outside an incubator cabinet, a system comprising the bioreactor and the gas-exchange unit, and a method of culturing a sample.

### FIELD OF THE INVENTION

The present invention relates to the field of biotechnology, especially to cell culture and tissue culture, more specifically to the long-term culturing of thick as well as small tissue samples in a system and further allowing for ex vivo live cell imaging.

### BACKGROUND

Cells rely on continuous supply with nutrients for survival. Multicellular organisms maintain their individual cells by performing such supply in many ways, for example, by blood circulation and diffusion of nutrients from the blood into the tissues, where cells can take up those nutrients. Cells or tissues separated from its organism can only be maintained under artificial nutrient supply. Such artificial nutrient supply is especially challenging for tissues, since here the cells are tightly packed at high density and thus represent a strong diffusion barrier.

Intravital live cell imaging visualizes cellular behavior with high spatial and temporal resolution. It is an in vivo technique preserving cells in their native environment, thus minimizing interference of external influences with their behavior. Therefore, it has become an important tool in medical sciences. However, it is incompatible with humans, on the sole reason that it requires a transparent surgically implanted imaging window. Further, not only for humans but also for animals, the imaging time is short due to the limited time of anesthesia acceptable, the throughput of samples is low and the access to the sample is limited. Thus, science relies on animal experiments with limited comparability and translatability into the human.

For reduction of animal experiments as well as for allowing such techniques to be applied directly to human cells or tissues, performing ex vivo experiments in a bioreactor has gained much interest in the past years. Bioreactors providing for nutrient supply of cells or tissues by superficial flow of a nutrient containing liquid and diffusion of nutrients into the cells were developed. Also, more advanced techniques aiming a perfusion of a tissue sample or cell-aggregates are known. However, providing a flexibly applicable bioreactor, suitable for thick and thin tissue samples, and allowing for live cell imaging imposes several challenges.

WO2014172575A1 discloses a bioreactor-system for tissue culture compatible with CT or NMR imaging. The tissue is perforated by channels that are circulated by medium such that nutrients may diffuse into the tissue. However, such system is not for use with standard cell culture equipment and the inner tissue structure is heavily impacted by such perforation. Furthermore, the imaging techniques are restricted to expensive, highly complex ones that are not available in standard cell culture laboratories and do not allow for imaging of fluorescence-labelled cells or structures, which is the most widely used technique for cellular imaging.

US20220033769A1 and WO2013182574A1 disclose systems comprising a bioreactor for cell and tissue culture suitable for culturing of biopsies. However, the bioreactor is not suitable for imaging of both thin and thick tissue samples, since especially thin samples would either be oriented such that an imaging or an efficient perfusion was impossible, since the inlet and the outlet of the sample-containing chamber are both located approximately in an 90° angle to the imaging plane.

WO2017025620 discloses a bioreactor for culturing artificial bioprinted tissues, comprising a movable element to regulate the volume of a sample-chamber, into which multiple channels, i.e., inlets and outlets, open. However, the channels need to be aligned to vessels in natural organs or to a network of artificial channels in artificial tissues. Albeit it is mentioned to culture natural tissue samples, in this case it is even more challenging to align natural vessels to the channels.

Thus, it is an object of the present invention to provide an improved system with which the disadvantages of the prior art are eliminated or at least mitigated. Especially, such a system should be provided which is suitable for long-term culturing of thin samples as well as thick samples and which allows for live cell imaging. This object is fully solved by the present invention.

### SUMMARY OF THE INVENTION

In aspect of the invention, the above identified disadvantages and others are overcome by providing a bioreactor for culturing a sample comprising tissue or embedded cells, said bioreactor comprising an inlet configured to introduce a fluid into the bioreactor, said inlet comprising
a first section comprising a sample bed, said sample bed being configured to hold the sample in a sample space, in which the sample is located, when the sample was inserted,
a second section being movable relative to the sample bed to compress the sample in between the second section and the sample bed, when the sample has been inserted, and being openable as to comprise a through-hole having a diameter, said through-hole being open towards the center of the sample space and configured for guiding a fluid-flow onto the center of the sample space, and
a third section configured to be connected to a fluid-supply.

According to the invention, the bioreactor is understood, as generally in the field, as comprising at least one inlet and at least one outlet for connecting the bioreactor to a fluid-supply and a fluid-discharge, for example, a tubing. Thus, the bioreactor may comprise an inlet as defined in claim 1 as well as further inlets. The terms "inlet" and "outlet" have to be understood as referring to means that can be embodied substantially identical. Thus, an outlet may also be used as an inlet or vice versa.

A "section" of the bioreactor may refer to a component of the bioreactor or to an arbitrarily defined region of a component comprising at least one section of the bioreactor. A component can be configured to be decomposed and thus removed from the bioreactor. An arbitrarily defined region of a component is not to be removed from the bioreactor or can only be removed in conjunction with at least one other section or region.

The sample can be tissue, embedded tissue, or embedded cells. The term "embedded" is to be understood such that a piece of tissue or cells are enclosed in a three-dimensional scaffold suitable to provide a degree of stability. Preferably the scaffold is a gel to be poured over or around the piece of tissue or the cells in a liquid state, after which it solidifies to form the gel. More preferably the scaffold is a collagen-hydrogel. A collagen-hydrogel advantageously is comparable to the natural extracellular matrix.

Preferably the scaffold is transparent. This advantageously allows the embedded tissue or embedded cells to be visually observed.

Preferably the tissue or embedded tissue is a piece of liver or ovary, more preferably a liver biopsy or an ovary biopsy. Those tissues have proven to be suitable for use with the bioreactor according to the invention.

Throughout herein, "openability" of the second section is to be understood such that the second section is "changeable" between an opened state and a closed state. The change can be achieved for example by moving, deforming, stretching, or by removing and replacing at least a part of the second section, or by introducing or removing a subject into or from the through-hole, which subject blocks said through-hole.

The openability of the second section is advantageous since it allows to culture thick and thin samples in the bioreactor in a perfusion or over-flow manner.

The term "perfusion" means that a fluid passes the inner of a tissue, which tissue may be embedded, or the inner of a scaffold into which cells are embedded.

The second section is further configured to compress the sample. Compression of the sample is advantageous for culturing in a perfusion manner, since the fluid-flow is forced this way to pass fully or almost fully through the sample. Furthermore, the sample is prohibited from being transported away by the fluid-flow.

The through-hole is configured for guiding a fluid-flow onto the center of the sample space. It will be clear to the skilled person that for this purpose the third section is in fluid communication at least with the through-hole and the sample bed.

When the second section is closed, a sample can be cultured by guiding a fluid-flow via an outlet over or through the sample. The sample in the sample space can be over-flown or perfused. The second section is preferably closed for culturing in an over-flow manner. For this purpose, the sample is preferably not compressed by the second section. Advantageously, this ensures that the fluid-flow over-flows the sample.

When the second section is opened, a sample can be cultured by guiding a fluid-flow via the through-hole over or through the sample. The sample in the sample space can be over-flown or perfused. The second section is preferably opened for culturing in a perfusion manner. For this purpose, the sample is preferably compressed. Advantageously, this ensures that the fluid-flow perfuses the sample.

In a preferred embodiment, the through-hole is openable by exchanging a closed second section by an opened second section.

In another preferred embodiment, the through-hole is openable by removing a plug or a stopper from the through-hole. This advantageously allows an easy opening of the through-hole.

In a preferred embodiment, the through-hole is changeable between
an inverted-funnel-shaped state, such that the through-hole comprises a narrowest section and a conical section and such that the sample space is cone-shaped wherein the conical section forms the tip of the cone-shaped sample space, and
a cylindrical-shaped state, wherein the diameter of the through-hole in the cylindrical-shaped state is wider than the diameter at the narrowest section of the through-hole in the inverted-funnel-shaped state.

The conical section and the cone-shaped sample space are understood to be substantially conical in shape, which means that they do not have to be exactly conical. Especially the sample space can deviate from the conical shape in that it comprises a substantially conical tip on top of a rather cylindrical or differently shaped part.

The inverted-funnel-shaped state causing a cone-shape of the sample space is advantageous for culturing of thick samples having a thickness of at least 1 mm, since the fluid-flow impinges due to the cone-shape of the sample space onto a reduced, focused, central area of the sample, thus maximizing the perfusion of the inner of the sample. Due to the thickness of the sample, the risk of disrupting the sample is limited. The perfusion is intensified especially in the center of the sample. For example, the sample can have a thickness of 1 mm, 2 mm, 3 mm, 4 mm or 5 mm, preferably the sample has a thickness of 2 mm.

The cylindrical-shaped state is advantageous for culturing of thin samples having a thickness of up to 1 mm, since the fluid-flow evenly impinges onto a large area of the sample, thus minimizing the risk of disrupting the sample. Due to the limited thickness of the sample, the fluid-flow can still sufficiently enter the inner of the whole sample.

In a preferred embodiment, the through-hole is changeable by exchanging an opened second section in the inverted-funnel-shaped state by an opened second section in a cylindrical-shaped state or vice versa.

In a preferred embodiment, the through-hole of the inverted-funnel-shaped state can be dumbbell-shaped.

In another preferred embodiment, at least one opening is formed between the second section and the sample bed, said opening being arranged circumferentially surrounding the sample space.

At least one opening "being arranged circumferentially surrounding the sample space" is to be understood such that the at least one opening leaves at least 60% of the circumference of the sample space open, more preferably 75%, further preferably 90%.

The at least one opening being arranged circumferentially surrounding the sample space is advantageous in that such opening allows the fluid-flow to uniformly exit the sample space in multiple or all directions.

When a single opening is provided, the fluid-flow can enter the sample space from one direction and exit to another direction through the same opening. When more than one opening is provided, the fluid-flow can enter the sample space from one direction and exit to another direction through the same or another opening.

When the second section is opened, the fluid-flow can enter the sample space via the through-hole and exit uniformly in multiple or all directions through the at least one opening being arranged circumferentially surrounding the sample space.

When the second section is closed, the fluid-flow can enter the sample space via the at least one opening from a first direction and exit the sample space to a second direction. Preferably the first and the second direction are parallel to each other, and the fluid-flow enters on a first side of the sample space and exits to a second, opposed side of the sample space.

In a preferred embodiment the at least one opening is a single ring-like opening. The ring-like opening advantageously allows the fluid-flow to exit the sample space in all directions.

In a preferred embodiment the at least one opening are two separated sickle-shaped openings.

The sickle-shaped openings advantageously allow the fluid-flow to exit the sample space in almost all directions. Furthermore, the sickle-shaped openings are especially advantageous when the second section is closed and the fluid is introduced via an outlet, since passage of the fluid-flow aside of the sample is blocked.

In another preferred embodiment, when the second section is opened, the channel is straight and has a center axis. The center axis is most preferably angled to the at least one opening circumferentially surrounding the sample space in an angle α of 90°.

In another preferred embodiment, the first section comprises a first hollow cylindrical body comprising a first inner thread located on the inner surface of the first hollow cylindrical body, and wherein the second section comprises a first outer thread that can be screwed into the first inner thread, wherein the second section is movable by screwing it in or out of the first inner thread.

This advantageously allows to keep the second section movable by screwing and at the same time openable by exchanging. Further advantageously, the degree of compression of the sample can be easily controlled by screwing the second section into the first hollow cylindrical body. It further advantageously allows easy access to the sample bed to clean it or to insert a sample, when the second section is removed from the first hollow cylindrical body.

In this embodiment, the second section and the third section are preferably one component.

In another preferred embodiment, the first hollow cylindrical body comprises a second outer thread located on the outer surface of the first hollow cylindrical body, and the third section comprises a second inner thread that can be screwed onto the second outer thread.

This advantageously allows to easily bring the third section into fluid communication with the through-hole. It further advantageously allows easy access to the sample bed to clean it or to insert a sample, when the second section and the third section are removed from the first hollow cylindrical body.

In another preferred embodiment, the sample bed is at least partially transparent such that the sample can be visually observed.

A visual observation can be for example an observation of the sample by eye or via a magnifying optical system as for example a microscope. It will be clear to the skilled person, that the sample can be observed in this or other embodiments with or without a partially transparent sample bed by imaging techniques as for example NMR or CT.

Transparency of the sample bed advantageously allows to visually observe the sample, in particular to image the sample. More advantageously, the sample can be observed during culturing and thus ex vivo live cell imaging using fluorescent cellular markers is possible. The use of live cell imaging using fluorescent cellular markers is advantageous since it allows high spatial and temporal resolution that allows visualization and tracking of single cells and subcellular compartments and it is a widely established technique that is relatively easy to be performed in a laboratory with standard-equipment. Equipment for NMR or CT is not considered to be standard laboratory equipment. Furthermore, fluorescent cellular markers allow to specifically visualize and identify a huge variety of different parts of a tissue or a cell using multiple cell-, compartment- or protein-specific markers at the same time.

In this embodiment, compression of the sample between the second section and the sample bed is further advantageous for allowing imaging of the sample, since this way the sample does not move in the fluid-flow and can be kept stationary in the object plane of a potential imaging unit observing the sample from outside the bioreactor.

In this embodiment, furthermore, the invention advantageously allows visual observation of both thin and thick samples. In embodiments in which the at least one opening is circumferentially surrounding the sample space, especially when the at least one opening is a single ring-like opening or two separated sickle-shaped openings, a field of view of an imaging unit is not blocked, hindered, or otherwise impaired by means for discharging the fluid passing the at least one opening, as for example an outlet or tubing. Bioreactors from prior art hardly allow for live cell imaging especially of thin samples, since the inlet and the outlet of the sample space are both located approximately in an 90° angle to the imaging plane.

The at least partially transparent sample bed can be from glass or plastic and is preferably a plane glass, more preferably a microscopical cover slip. This advantageously allows to easily perform microscopy on the living sample.

In another preferred embodiment, the bioreactor comprises a mesh, wherein the mesh is located in between the sample bed and the second section and wherein the sample, when inserted, is enclosed between the mesh and the sample bed.

The mesh advantageously allows to immobilize the sample on the sample bed. A slipping of the sample by chance, a moving of the sample, especially an uncompressed sample, by the fluid-flow, or a shunting of the sample during compression is minimized. At the same time easy perfusion or diffusion of the fluid through the sample is ensured by embodying this immobilization means as a mesh.

In another preferred embodiment, the bioreactor is produced by 3D-printing. This allows individualized production, potentially according to the needs of the respective user, such that the bioreactor can be fitted to the specific needs of the sample to be cultured.

In another preferred embodiment, the material from which the bioreactor is made is biocompatible, preferably a biocompatible medical resin. A washout of ingredients or substances from the material by the fluid-flow should be minimized, since those would get into contact with the sample. Such choice of the bioreactor's material advantageously avoids negative influences of the bioreactor's material onto the sample to be cultured.

In another preferred embodiment, the material from which the bioreactor is made is autoclavable. This advantageously allows to sterilise and to reuse the bioreactor.

In another preferred embodiment, the bioreactor can comprise plugs to be inserted into openings of the bioreactor for autoclaving. Such plugs advantageously ensure sterility of the inner of the bioreactor after autoclaving and before use. Preferably plugs for all openings representing a connection to the inner of the bioreactor during autoclaving are provided.

In another preferred embodiment, the second section can be configured to be screwed by a screwdriver, which advantageously allows easy assembly and disassembly of the bioreactor. Further, a stand for the screwdriver can be provided, which advantageously allows a safe, easy storage of the screwdriver without necessity to lay it down to a working surface, which might impair its sterility.

In another aspect, the invention relates to a gas-exchange unit for allowing gas-exchange of a cell culture medium or a tissue culture medium during cell culture or tissue culture, the gas-exchange unit comprising
a main body comprising
   a tubular lumen,
   a first connector, configured to be connected to a vessel, and allowing free passage of gases,
   a second connector, configured to be at least intermediary connected to a gas-supply, and allowing free passage of gases, and
a gas-filter membrane,
wherein the gas-filter membrane is not permeable for microorganisms, is permeable for gases, and spans the tubular lumen between the first and the second connector such that a gas passing the tubular lumen from the second to the first connector passes the gas-filter membrane.

According to the invention, the vessel the first connector is configured to be connected to contains a fluid to be circulated through the bioreactor. Such fluid is preferably a liquid, further preferably cell culture medium or tissue culture medium, both of which are water-based liquids supplemented with at least nutrients for the respective tissue or cells.

Throughout this specification, the term "fluid-system" refers to a volume extending through at least one outlet, the sample space, and the inner volume of the vessel the first connector is configured to be connected to. The volume preferably further extends through a tubing interconnecting the bioreactor and the gas-exchange unit, a pump and through the inlet. The fluid to be circulated through the bioreactor is also to be circulated through the fluid-system and will be referred to as the fluid to be circulated.

Throughout this specification the term "gas volume" refers to a volume excluded from the fluid-system and extending adjacent to a side of the gas-filter membrane not facing the fluid-system.

The "tubular" lumen is to be understood as an inner volume of the main body, into which a gas can enter from and exit to the outside of the main body on at least two distinct positions of the main body.

A "connector" is to be understood as a structure comprising means for connecting two subjects releasably. Examples of connectors are threads, click-connections, plug-connections, or pneumatic connections.

The releasability of the first connector advantageously allows to easily change the vessel the first connector is configured to be connected to. Furthermore, easy access to the fluid to be circulated is provided such that it can be easily changed, supplemented, filled up, removed or the like.

The releasability of the second connector advantageously allows to keep the gas-exchange unit in an incubator cabinet or outside such cabinet and connected to a gas-supply.

The gas-exchange unit advantageously allows a regulated exchange of gases between the fluid to be circulated through and the gas volume. Especially, the CO₂ and the O₂ level inside the fluid-system has to be regulated, for example to maintain a desired pH value and to allow cellular respiration, respectively.

Further, the gas-exchange unit allows such gas-exchange to take place in an incubator cabinet as well as outside such cabinet. Usually, cell culture flasks, tissue culture flasks or bioreactors are kept in a CO₂ atmosphere of for example 5 % v/v inside incubator cabinets, which are standard cell or tissue culture equipment and provide such atmosphere. Outside such atmosphere, i.e., outside an incubator cabinet, evaporation of CO₂ from the cell culture medium or tissue culture medium takes place, causing a raising pH value. The gas-exchange unit according to the invention advantageously allows to be connected to a gas-supply such that it can be kept outside an incubator cabinet for an extended period while prohibiting an undesired change in the pH value of the medium.

The gas-filter membrane is gas-permeable, microorganism-impermeable, and spans the tubular lumen between the first and the second connector such that a gas passing the tubular lumen from the second to the first connector passes the gas-filter membrane. Thus, advantageously, any gas entering the vessel (i.e., the fluid-system), when the vessel is connected to the first connector, is sterile-filtered. This advantageously keeps the fluid-system and the sample sterile, while gases can be exchanged between the fluid-system and the gas volume. It will be clear to the skilled person that, for this purpose, the gas-filter membrane is to be fixated along all its edges in a microorganism-tight manner to, for example, the main body.

In a preferred embodiment such fixation is achieved by attaching all edges of the gas-filter membrane to the main body, for example by gluing, plastic-welding, or clamping, most preferably by clamping. Clamping advantageously allows to replace the gas-filter membrane when necessary.

In another preferred embodiment, the gas-exchange unit comprises an inlet-tube configured to introduce the fluid to be circulated into the vessel the first connector is configured to be connected to and an outlet-tube configured to discharge said fluid from said vessel. This advantageously allows connection of the gas-exchange unit to the fluid-system.

In a preferred embodiment, the inlet-tube extends through a plane of the gas-filter membrane and the second connector, and the outlet-tube extends from a bottom of the vessel the first connector is configured to be connected to, through a plane of the gas-filter membrane, and the second connector. Advantageously, when being introduced, the fluid drops or falls from the inlet-tube down towards the bottom of said vessel. Without being bound to the theory, it is assumed that this enhances the gas-exchange between the fluid and the gas volume. The outlet-tube advantageously extends from the bottom of said vessel, which prevents any gas located over the fluid contained in said vessel to be discharged from said vessel, for example, into the bioreactor. This advantageously allows to pump or suck the fluid to be circulated out of said vessel and through the fluid-system. Further advantageously, in this embodiment the gas-exchange unit also serves as a bubble-trap, which allows for a steady fluid-flow.

In a preferred embodiment, the gas-exchange unit comprises a counter bridge comprising a first fluid-pass and a second fluid-pass, each fluid-pass extending through the plane of the gas-filter membrane or through the plane of the gas-filter membrane and through the second connector, and an uptake-tube extending from the second fluid-pass to the bottom of the vessel the first connector is configured to be connected to, wherein the inlet-tube is connected to the first fluid-pass and extends inside the second connector and the outlet-tube is connected to the second fluid-pass and extends inside the second connector. In this embodiment, the inlet-tube and the outlet-tube do not extend through the plane of the gas-filter membrane, which advantageously facilitates connecting and disconnecting the inlet-tube and the outlet-tube to the gas-exchange unit, since they do not have to be plugged through and fixated with the gas-filter membrane. Furthermore, advantageously the inlet-tube and the outlet-tube do not have to stay permanently fixated to the gas-filter membrane, which facilitates replacement and/or cleaning.

In a preferred embodiment, the inlet-tube and the outlet-tube extend each through the second connector by extending through a tubing hole in the second connector or a recess at an edge of the second connector.

In a preferred embodiment, the inlet-tube and the outlet-tube or the first fluid-pass and the second fluid-pass extend each through the plane of the gas-filter membrane by extending through a fenestration in the second connector or a recess at an edge of the gas-filter membrane.

In a further preferred embodiment, the first connector comprises a thread, preferably a third inner thread, such that the first connector is configured to be screwed onto a threaded vessel.

The first connector comprising a thread advantageously allows the gas-exchange unit to be connected to a vessel otherwise closed by a threaded lid, which is a widely used option for closing a vessel.

Preferably the vessel the first connector is configured to be connected to is a standard laboratory-grade plastic vessel, for example a centrifuge vessel. Such vessels are usually closed by a threaded lid. Advantageously, this makes the gas-exchange unit compatible with standard laboratory equipment and thus flexible in use. Moreover, the vessel can be easily and flexibly changed, supplemented, filled up, removed or the like.

In another preferred embodiment the second connector is configured to be intermediary connected to a gas-supply.

An "intermediary" connection of the second connector is to be understood such that the second connector is connected to a component comprising a third connector to be directly connected to a gas-supply. A "direct" connection is to be understood such that a connector is itself connected to a gas-supply.

In another preferred embodiment, the gas-exchange unit comprises a lid configured to be connected to the second connector, said lid comprising a third connector configured to be connected directly to the gas-supply. Thus, in this embodiment the second connector is configured to be connected intermediary to a gas-supply via the lid.

Such lid is especially advantageous when the second connector or the third connector does not allow free passage of gases or limits the passage of gases due to, for example, a small diameter for free gas passage, when the respective connector is not connected to the gas-supply. Advantageously the lid ensures free gas passage between the gas volume and the surrounding of the gas-exchange unit, when removed from the second connector.

In a preferred embodiment, the third connector preferably comprises a pneumatic connector.

The third connector comprising a pneumatic connector advantageously allows to easily connect the gas-exchange unit to, for example, a pipe of a gas-supply, while said pipe is under pressure. The gas-supply is preferably the gas-supply of a microscope stage top environmental chamber, which enhances compatibility with a microscope stage top incubator.

In another preferred embodiment the second connector is configured to be directly connected to a gas-supply.

The second connector to be directly connected to a gas-supply advantageously reduces the number of components the gas-exchange unit comprises. This facilitates the handling.

In preferred embodiments, the second connector comprises a thread, preferably a third outer thread, such that the second connector is configured to be connected to the gas-supply intermediary or directly via a screw connection.

The second connector comprising a thread advantageously allows a quick, easy, and stable connection between the gas-exchange unit and the gas-supply.

In preferred embodiments, the main body and/or the lid have a grippy profile on their outer surface. This advantageously facilitates the handling, especially the connecting of the connectors.

In further preferred embodiments, at least the main body and, in case provided, the lid of the gas-exchange unit is produced by 3D-printing. 3D-printing allows individualized production, potentially by the respective user of the bioreactor, such that the gas-exchange unit can be fitted to the specific needs of the fluid-system, in which the culture medium is contained.

In further preferred embodiments, the material from which at least the main body and, in case provided, the lid of the gas-exchange unit is made is biocompatible, preferably the material is a biocompatible medical resin. A washout of ingredients or substances from the material by the fluid-flow should be minimized, since those would get into contact with the sample. Such choice of the bioreactor's material advantageously avoids negative influences of the bioreactor's material onto the sample to be cultured.

In another preferred embodiment, the material from which at least the main body and, in case provided, the lid of the gas-exchange unit is made is autoclavable. This advantageously allows to sterilise and to reuse the gas-exchange unit.

The invention further relates to a system for culturing a sample comprising tissue or embedded cells, the system comprising
at least one bioreactor,
at least one gas-exchange unit, and
a tubing comprising a pump.

The at least one bioreactor may be a bioreactor according to the invention, and/or the at least one gas-exchange unit may be a gas-exchange unit according to the invention.

The tubing can be made from rubber, preferably from silicone. The tubing can comprise a plurality of pipes (or tubes) having a wall with a wall-thickness and an inner diameter, said wall defining an inner volume of the pipe being configured to allow a fluid to pass. Said inner diameter can be up to 10 mm, preferably the inner diameter ranges from 1 mm to 6 mm, more preferably from 2 mm to 4 mm.

Preferably the pump is a peristaltic pump. A peristaltic pump advantageously provides an even and continuous fluid circulation without the necessity of valves. It further allows to keep the fluid-system closed, i.e., fully separated from the gas volume, the surrounding, or parts of the pump, to keep at least the fluid and the sample sterile.

In a preferred embodiment,
the bioreactor and at least a part of the tubing are configured to be inserted together into an at least partially transparent microscope stage top incubator and the gas-exchange unit is configured to be inserted into a heatable water bath, and
the bioreactor, the gas exchange unit, and at least a part of the tubing are configured to be inserted together into an incubator cabinet.

Such configuration of the system is advantageous since it allows to operate and keep the system inside an incubator cabinet as well as outside such cabinet for an extended period. To keep the system inside a cabinet advantageously allows to store it, for example, overnight or when an observation of the sample is not desired. To keep the system outside such cabinet advantageously allows to supervise the function of the system, to manipulate the fluid and/or the sample, and/or to observe the sample visually.

To keep the bioreactor inside a microscope stage top incubator advantageously allows to heat the bioreactor, for example to 37°C, while being kept outside an incubator cabinet.

In a preferred embodiment, the system further comprises a rack configured to hold the vessel the first connector of the gas-exchange unit is configured to be connected to. This advantageously allows to place the respective vessel upright on a flat surface, as for example the working-surface or the bottom of the heatable water bath. More preferably, the rack is configured to be placed together with the respective vessel into the heatable water bath.

In another preferred embodiment, the system comprises two bioreactors. This advantageously allows to culture two identical or different samples at the same time under the same or different conditions.

In this embodiment, the sample bed of each bioreactor is preferably at least partially transparent. This advantageously allows to simultaneously visually observe and/or image two samples. For example, one sample can serve as a control and the other one is cultured under conditions, whose effect onto the sample shall be investigated.

In a preferred embodiment, the system further comprises an imaging unit configured for imaging the sample.

The imaging unit can comprise a device for optical magnification of the sample, for example a microscope. This advantageously allows to observe the sample not only macroscopically, but also on a microscopic level. For example, the microscopic structure of a sample, or the behavior or properties of cells can be observed. The device for optical magnification can be for example a fluorescence microscope, a total internal reflection microscope, a confocal microscope, a two-photon microscope, a multi-photon microscope, or a light-sheet microscope. Preferably the imaging unit comprises a multi-photon microscope, which advantageously allows to image the sample on cellular and/or subcellular level in a depth of up to more than 1 mm.

Further, the imaging unit can comprise means for imaging the sample, i.e., for acquiring pictures or videos of the sample. The means for imaging the sample can allow for any imaging technique, as for example CT, NMR, or live cell imaging with a microscope.

Preferably, the imaging unit comprises means allowing for live cell imaging of the sample. Live cell imaging advantageously allows imaging and observation of living samples and thus spatiotemporal resolution of, for example the behavior of cells or tissue under certain conditions. Especially when culturing a tissue sample in a bioreactor, live cell imaging allows to observe and image the behavior or properties of the cells in the tissue in their native environment.

Most preferably the live cell imaging can be performed during culturing of the sample. This advantageously allows to continue the culturing during and after the imaging. Thus, the sample can be imaged repeatedly at different durations of the culturing and/or sequentially, for example under different conditions. Advantageously a necessity for discontinuing or pausing the culturing and/or for removing the sample from the bioreactor for imaging is avoided.

The invention further relates to a method for culturing a sample having a thickness of more than 1 mm comprising tissue or embedded cells, said method comprising the following steps:
immobilizing the sample at a base of a cone-shape sample space,
focusing a fluid-flow towards the center of the sample by guiding the fluid-flow via the tip of the cone-shaped sample space into the sample and allowing the fluid-flow to exit the sample via a rim of the base of the cone-shaped sample space, and
maintaining the sample for a desired period.

As the inventors realized, reliable perfusion of samples having a thickness of at least 1 mm requires an enhancement of the perfusion especially in the center of the sample, which is less accessible to culture medium as compared to, for example, the rims of the sample.

Immobilizing the sample at a base of a cone-shape sample space and focusing a fluid-flow through the tip of the cone-shaped sample space is advantageous in that allows to perfuse the sample and especially its center.

The perfusion is sustained by allowing the fluid-flow to exit the sample via the rim of the base of the cone-shaped sample space. Since the fluid-flow exits the sample at the rim of the base, it has to perfuse a large part if not all of the sample.

In another embodiment, the method comprises the use of a bioreactor.

In another embodiment, the method comprises the use of a bioreactor according to the invention or a system according to the invention.

The use of the bioreactor or the system according to the invention advantageously allows the culturing of thick samples having a thickness of at least 1 mm, as well as the culturing of thinner samples. For example, the sample can have a thickness of 1 mm, 2 mm, 3 mm, 4 mm or 5 mm, preferably the sample has a thickness of 2 mm.

In another preferred embodiment, the method further comprises the step of imaging the sample when the system comprises the imaging unit. Equivalent advantages that apply to the system comprising an imaging unit apply to the method comprising an imaging step.

It is understood that the features mentioned above and those yet to be explained below can be used not only in the particular combination given, but also in other combinations or in isolation, without departing from the scope of the present invention. Especially, the features, characteristics, embodiments, and advantages mentioned with respect to the bioreactor similarly apply to the system comprising such bioreactor.

The invention is now described and explained in further detail by referring to the following non-limiting examples and drawings.

### BRIEF DESCRIPTION OF THE DRAWINGS

In the drawings:
Fig. 1A shows a scheme of a bioreactor for culturing a sample comprising tissue or embedded cells comprising a closed second section;
Fig. 1B shows a sketch of a bioreactor for culturing a sample comprising tissue or embedded cells comprising an opened second section;
Fig. 1C shows a sketch of a second section of the bioreactor in the inverted-funnel-shaped state;
Fig. 2 shows a bioreactor for culturing a sample comprising tissue or embedded cells;
Fig. 3 shows the second section of the bioreactor for culturing a sample comprising tissue or embedded cells.
Fig. 4 shows a bioreactor for culturing a sample comprising tissue or embedded cells as a section view.
Fig. 5A shows a gas-exchange unit for allowing gas-exchange of a cell culture medium or a tissue culture medium during cell culture or tissue culture.
Fig. 5B shows a second compression ring of a counter bridge of a gas-exchange unit as a top view.
Fig. 6 shows a gas-exchange unit for allowing gas-exchange of a cell culture medium or a tissue culture medium during cell culture or tissue culture as a section view.
Fig. 7A shows a scheme of a system for culturing a sample comprising tissue or embedded cells, comprising a bioreactor comprising an opened second section.
Fig. 7B shows a scheme of a system for culturing a sample comprising tissue or embedded cells comprising a bioreactor comprising a closed second section.
Fig. 8 shows a simulation of the fluid-flow through the through-hole in the inverted-funnel-shaped state of a second section of a bioreactor.
Fig. 9A shows a simulation of the fluid-flow through the sample space of a bioreactor when the through-hole is in a cylindrical-shaped state or in the inverted-funnel-shaped state as a top view onto the sample space.
Fig. 9B shows a visualization of the DRAQ5-concentration as a measure of the fluid-flow through the sample space of a bioreactor when the through-hole is in a cylindrical-shaped state or in the inverted-funnel-shaped state as a top view onto the sample space.
Fig. 10A shows a diagram showing the total number of cleaved caspase-3 positive cells in liver biopsies, normalized to the overall tissue area for a top tissue section, a middle tissue section, and a bottom tissue section after two days of culture.
Fig. 10B shows a spatial distribution of cleaved caspase-3 positive cells in liver biopsies, distributed into 40 × 40 bins for a top tissue section, a middle tissue section, and a bottom tissue section after two days of culture.
Fig. 11 shows a time projection from a 30-minute video acquired during culture of an ovarian biopsy after 4 days of culture.

### DETAILED DESCRIPTION OF PREFERRED EMBODIMENTS

### 1. Bioreactor of the invention

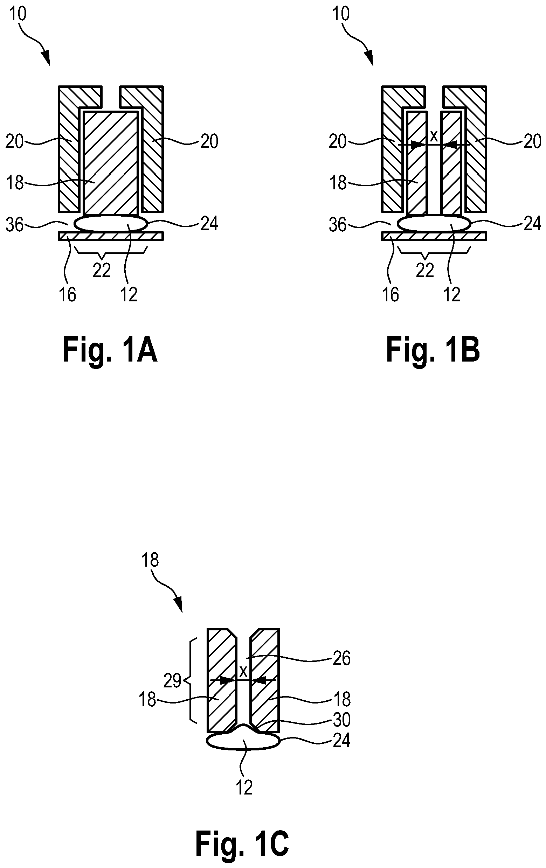

Fig. 1A and 1B show a sketch of a bioreactor 10 for culturing a sample 12, comprising an inlet configured to introduce a fluid into the bioreactor 10, said inlet comprising a first section 16, a second section 18 and a third section 20.

The first section 16 comprises a sample bed 22, said sample bed 22 being configured to hold the sample 12 in a sample space 24, in which the sample 12 is located.

The sample bed 22 can be a microscopical coverslip from glass having a thickness of 170 µm and a transparent area for visual observation of the sample can have a diameter of 10 mm.

The second section 18 is movable relative to the sample bed 22 to compress the sample 12 in between the second section 18 and the sample bed 22. The second section 18 is openable.

The third section 20 is configured to be connected to a fluid-supply.

Fig. 1A shows the bioreactor 10, wherein the second section 18 is closed.

Fig. 1B shows the bioreactor 10, wherein the second section 18 is opened as to comprise a through-hole 26 having a diameter x, said through-hole 26 being open towards the center of the sample space 24 and configured for guiding a fluid-flow onto the center of the sample space 24.

In a preferred embodiment, through-hole 26 has an inverted-funnel-shaped state, such that the through-hole 26 comprises a narrowest section 29 and a conical section 30 and such that the sample space 24 is cone-shaped wherein the conical section 30 forms the tip 32 of the cone-shaped sample space 24. The narrowest section 29 is the section comprising the lowest diameter x as compared to any other section of the through-hole 26 in the inverted-funnel-shaped state.

In a preferred embodiment, the through-hole 26 has a cylindrical-shaped state, wherein the diameter x of the through-hole 26 in the cylindrical-shaped state is wider than the diameter x at the narrowest section 29 of the through-hole 26 in the inverted-funnel-shaped state.

The bioreactor 10 further comprises at least one opening 36 formed between the second section 18 and the sample bed 22. The at least one opening 36 is arranged circumferentially surrounding the sample space 24 and allows the fluid-flow to exit or enter the sample space 24.

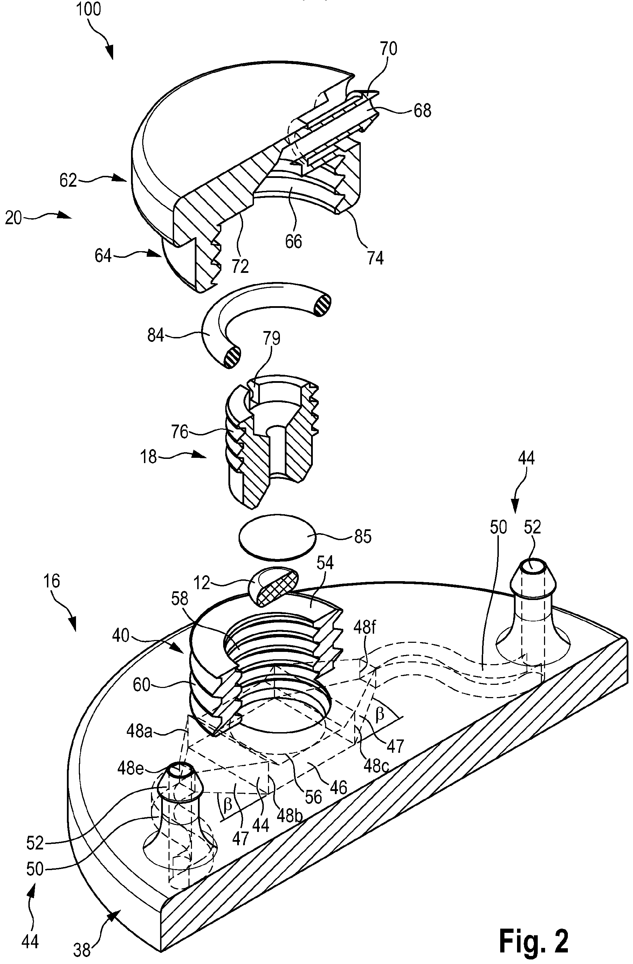

Fig. 2 shows a bioreactor 100. The bioreactor 100 comprises an inlet comprising a first section 16, a second section 18, and a third section 20. Features identical to the features shown in Fig. 1 are referred to with the same reference sign, respectively.

The first section 16 of the bioreactor 100 comprises a base 38 and a first hollow cylindrical body 40.

The base 38 can be a semicircular plate that may have a radius of 57 mm and include a hexagonal chamber 42 and two outlets 44. The chamber 42 can have a bottom 46 and six edges 48a, b, c, d, e, f. The bottom 46 of the chamber 42 can comprise the sample bed 22. The sample bed 22 can be a square area located in between the edges 48a, b, c, d. The bottom 46 can further comprise two areas 47 located in between the edges 48a, b, e and 48c, d, f, respectively. The areas 47 are obtusely angled relative to the sample bed 22 in an angle β as to taper the chamber 42 towards the outlets 44. The edges 48e and 48f can open into the outlets 44. The outlets 44 can comprise each a hollow channel 50 and a port 52, said hollow channels 50 being configured to guide the fluid-flow from the chamber 42 to the ports 52 or vice versa. The ports 52 are configured to be connected to a tubing (not shown), for example by being at least partially introduced into the ends of said tubing. Any outer edges of the base 38 can be rounded.

In other preferred embodiments, the plate forming the base 38 can be of any shape. For example, the plate can be circular, square, polygonal, oval, or irregular.

In other preferred embodiments, multiple bioreactors can be connected. For example, two semicircular plates forming bases 38 can be connected or be one piece, to form a circular multichannel-base 39. Such multichannel-base 39 may comprise more than two bases 38.

In the embodiment of bioreactor 100, the first hollow cylindrical body 40 can comprise a first open end 54, a second open end 56, a first inner thread 58 located on the inner surface of the first hollow cylindrical body 40, and a second outer thread 60 located on the outer surface of the first hollow cylindrical body 40. The first inner thread 58 can be an M10x1.5 thread. The second outer thread 60 can be an M16x2 thread. The first hollow cylindrical body 40 can open into the chamber 42 through the second open end 56.

The ports 52 are configured to be connected to a tubing (not shown) having an inner diameter ranging from 2 to 4 mm.

The third section 20 of the bioreactor 100 can comprise a plane circular body 62, a second hollow cylindrical body 64, a second inner thread 66, a hollow channel 68, and a port 70.

The plane circular body 62 can include the hollow channel 68 configured to guide the fluid-flow from the port 70 to the second hollow cylindrical body 64.

The second hollow cylindrical body 64 can comprise a first end 72, a second end 74, and the second inner thread 66 adjacent to the second end 74, and extends perpendicular from the plane circular body 62, said plane circular body 62 thus closing the first end 72 of the second hollow cylindrical body 64.

The second inner thread 66 can be an M16x2 thread and can be screwed onto the second outer thread 60 of the first hollow cylindrical body 40.

The port 70 is configured to be connected to a fluid-supply (not shown), preferably to a tubing for fluid-supply having an inner diameter ranging from 2 to 4 mm, for example, by being at least partially introduced into the end of said tubing.

The second section 18 of the bioreactor 100 can comprise a first outer thread 76 on its outer surface. The first outer thread 76 can be an M10x1.5 thread and can be screwed into the first inner thread 58, wherein the second section 18 is movable by screwing it in or out of the first inner thread 58.

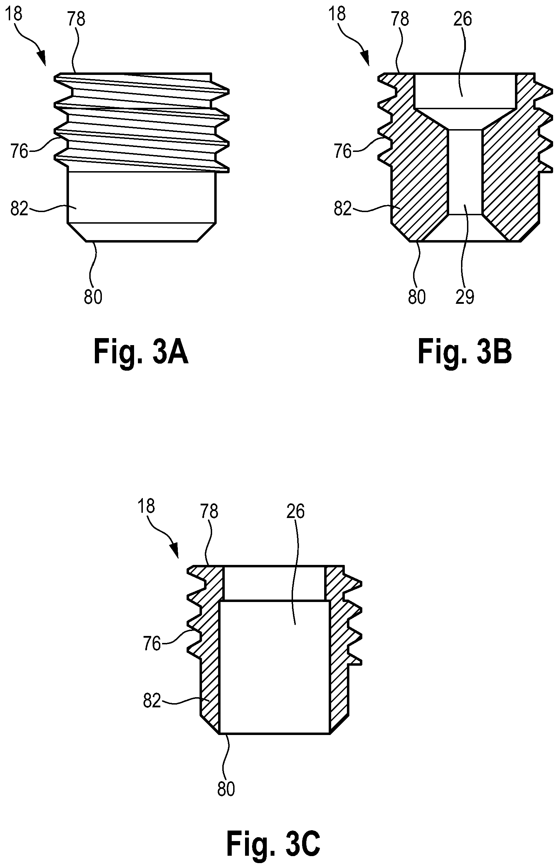

Fig. 3 shows three preferred embodiments of the second section 18. In all three embodiments, the second section can be overall cylindrical and comprise an inflow end 78, an outflow end 80, and a cylinder shell 82. The inflow end 78 is configured to let the fluid-flow enter the through-hole 26, in case the second section 18 is opened. The outflow end 80 is configured to let the fluid-flow exit the through-hole 26, in case the second section is opened. At the outflow end 80, the second section 18 may taper. The first outer thread 76 can cover approximately half of the cylinder shell 82 and be located adjacent to the inflow end 78.

The second section 18 can be configured to be screwed by a screwdriver (not shown). In a preferred embodiment, the second section 18 comprises two recesses 79 (shown in Fig. 2) at opposed sides of the inflow-end 78, which recesses are configured to provide grip for a screwdriver.

In other embodiments, the first outer thread 76 can cover from 20% to 100% of the cylinder shell 82.

Fig. 3A shows a preferred embodiment of the second section 18. In this embodiment, the second section 18 is closed, such that it does not comprise a through-hole 26.

Fig. 3B shows a preferred embodiment of the second section 18. In this embodiment, the second section 18 is opened as to comprise a through-hole 26. The through-hole 26 is in the inverted-funnel-shaped state, in which state the diameter x of the through-hole 26 can be 6 mm at the inflow-end 78, 5 mm at the outflow end 80, and 4 mm at the narrowest section 29 of the through-hole 26.

Fig. 3C shows a preferred embodiment of the second section 18. In this embodiment, the second section 18 is opened as to comprise a through-hole 26. The through-hole 26 is in the cylindrical-shaped state, in which state the through-hole 26 can have a diameter of 6 mm.

In the embodiment of bioreactor 100, the second section can be openable by and the through-hole 26 can be changeable by exchanging a second section 18 by a differently embodied second section 18.

In other preferred embodiments, the second section 18 can comprise a through-hole 26, which is obstructed when the second section 18 is closed. In such embodiment the closing can be achieved by a plug or stopper inserted into the through-hole 26. The opening can be achieved by removing such plug or stopper.

In other preferred embodiments, the second section 18 can be openable by and the through-hole 26 can be changeable by moving, deforming, or stretching at least a part of the second section 18.

The bioreactor 100 can further comprise a sealing ring 84 from silicone which may have an inner diameter of 9 mm and an outer diameter of 13 mm. The sealing ring 84 can be located in between the first section 16 and the third section 20, more specifically adjacent to the first open end 54 of the first hollow cylindrical body 40, and the plane circular body 60 and serves as a fluid-tight sealing between the first section 16 and the third section 20, when the second inner thread 66 is screwed onto the second outer thread 60.

In other embodiments, the sealing ring 84 can be attached to the first or the third section.

The bioreactor 100 can further comprise a mesh 85 that can be circular and can have a diameter of 8 mm and a mesh size of 100 µm. The mesh 85 is located in between the sample bed 22 and the second section 18. The sample 12, when inserted, is enclosed between the mesh 85 and the sample bed 22.

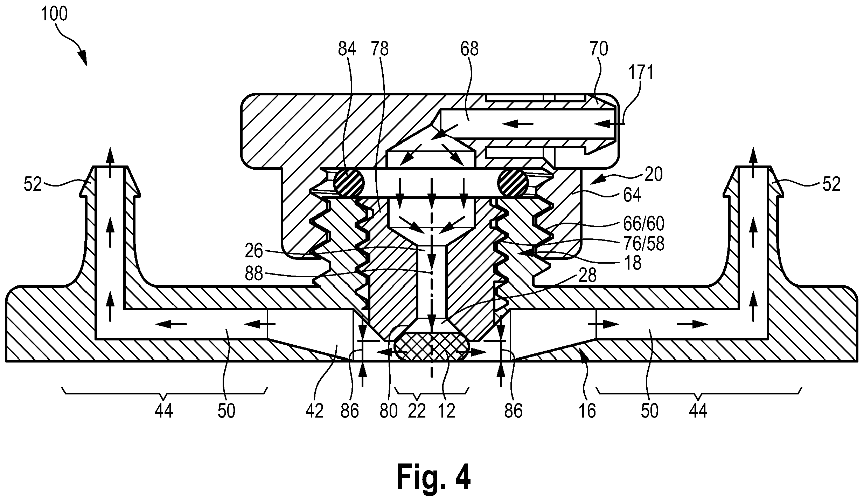

Fig. 4 shows bioreactor 100 for culturing a sample 12 comprising tissue or embedded cells in an assembled state as a section view. The second section 18 can be opened as to comprise a through-hole 26, which through-hole 26 can be in the inverted-funnel-shaped state, and the sample 12 can be placed onto the sample bed 22 in the chamber 42 of the first section 16.

The second section 18 can be screwed with the first outer thread 76 into the first inner thread 58 of the first hollow cylindrical body 40 such that the sample 12 can be compressed in between the first section 16 and the second section 18 and such that the at least one opening 36 can be formed as two sickle-shaped openings 86.

In other embodiments, the opening 36 can be for example a ring-shaped opening.

The third section 20 can be screwed with the second inner thread 66 onto the second outer thread 60 of the first hollow cylindrical body 40 and the sealing ring 84 can seal the bioreactor.

A fluid (not shown), preferably a liquid, more preferably tissue culture medium or cell culture medium, can be circulated in a fluid-flow (indicated by arrows 171; for the sake of clarity, not each arrow 171 is marked with the reference sign 171) through the bioreactor 100. The fluid can be introduced via the inlet. More specifically, first the fluid can be introduced via the port 70 into the hollow channel 68 guiding the fluid-flow from the port 70 to the second hollow cylindrical body 64.

Next, the fluid-flow may pass through the sealing ring 84 and enter the through-hole 26 of the second section 18 via the inflow end 78, pass the through-hole 26, and exit the through-hole 26 via the outflow end 80.

When exiting the through-hole 26, the fluid at least partially enters and passes the sample 12, thus perfuses the sample 12 (i.e., enters, passes, and thus perfuses the sample space 24). Further, the fluid may exit the sample 12 at the opening 36, pass said opening 36 and the chamber 42. Via the edges 48e and 48f of the chamber 42 the fluid-flow can be introduced into the outlets 44. More specifically, the fluid-flow can be introduced into the hollow channels 50 and be guided from the chamber 42 to the ports 52. Finally, the fluid-flow can exit the bioreactor 100 via said ports 52.

The through-hole 26 can have a center axis 88. The center axis 88 can be angled to the at least one opening 36 circumferentially surrounding the sample space 24 in an angle α of 90°.

In other embodiments, the angle α can be from 60° to 90°.

In other preferred embodiments, means for assembling the first section 16 and the second section 18 and/or means for assembling the first section 16 and the third section 20, embodied as thread connection in the embodiment of bioreactor 100, can be any other means allowing to keep the second section 18 movable relative to the sample bed. Examples are click-connections, sliding connections with locking screw, or magnetic connections.

In other preferred embodiments, at least the second section 18 and the third section 20 can be a single component.

### 2. Gas-exchange unit of the invention

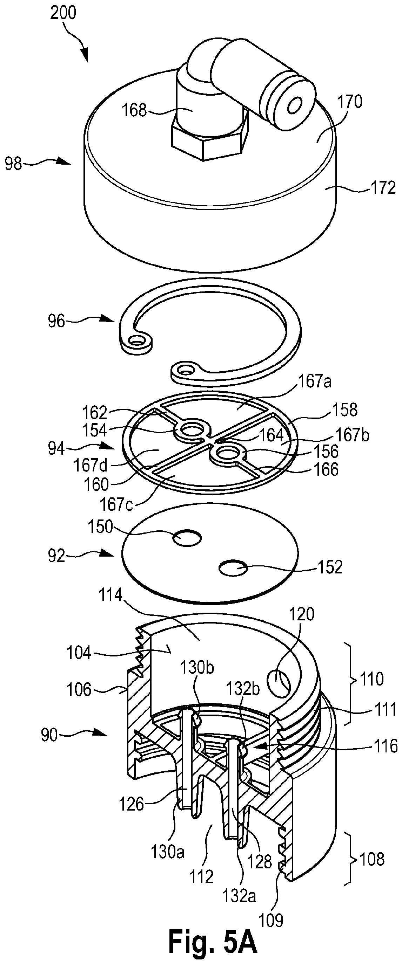

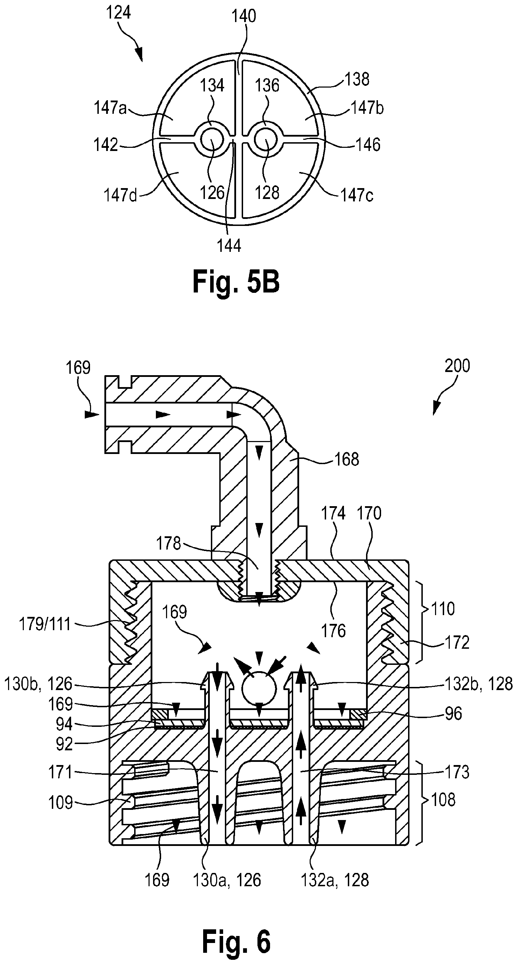

Fig. 5 shows a gas-exchange unit 200 for allowing gas-exchange of a cell culture medium or a tissue culture medium during cell culture or tissue culture. The gas-exchange unit 200 comprises a main body 90 and a a gas-filter membrane 92, and can comprise a first compression ring 94, a C-clip 96, a lid 98, an inlet-tube, (not shown), an outlet-tube (not shown), and an uptake-tube (not shown).

The main body 90 comprises a tubular lumen, a first connector 108, and a second connector 110 and can comprise an inner surface 104, an outer surface 106, a first connector-opening 112, a second connector-opening 114, a counter bridge 116, a first tubing hole 120, and a second tubing hole (not shown).

The main body 90 can be shaped approximately cylindrical and comprise a plurality of protrusions and recesses on the outer surface 106. Those processes and recesses advantageously form a grippy profile for facilitated handling of the gas-exchange unit.

The first connector 108 can comprise a third inner thread 109 located on the inner surface 104 adjacent to the first connector-opening 112 allowing free passage of gases through the first connector 108. The first connector 108 can be configured to be connected to a thread of a standard laboratory 50-mL centrifuge vessel (not shown). However, it is clear to the skilled person, that the vessel may have any volume, preferably at least a volume of 15 mL.

The second connector 110 can comprise a third outer thread 111 located on the outer surface 106 adjacent to the second connector-opening 114 allowing free passage of gases through the second connector 110. The third outer thread 111 can be an M32x2 thread. The second connector 110 can be configured to be at least intermediary connected to a gas-supply (not shown).

In other preferred embodiments, the first connector 108 and/or the second connector 110 can comprise any means for connecting two subjects releasably. Examples are threads, click-connections, plug-connections, or pneumatic connections.

The counter bridge 116 can comprise a second compression ring 124, a first fluid-pass 126, and a second fluid-pass 128 and can be located between the first connector 108 and the second connector 110.

The first fluid-pass 126 and the second fluid-pass 128 each may be a channel or tube comprising a first port 130a, 132a and a second port 130b, 132b for connection to a tubing. The first port 130a, 132a may extend from the second compression ring 124 into the first connector 108 and can be configured to be connected to a tubing, which tubing may have a diameter ranging from 4 to 6 mm. The second port 130b, 132b may extend into the second connector 110 and can be configured to be connected to a tubing, which tubing may have a diameter ranging from 2 to 4 mm. Both ports 130a, 132a, 130b, 132b can be connected to the tubing by at least partially introducing the respective port into an end of the tubing.

In other embodiments, the diameter of the ports 130a, 132a, 130b, 132b can range between 2 mm and 10 mm, depending on the specific tubing used.

Fig. 5B shows a top view onto the second compression ring 124. The second compression ring 124 can comprise a first ring 134, a second ring 136, and a third ring 138, a first strut 140, a second strut 142, a third strut 144, and a fourth strut 146, which rings and struts can be in a single plane such that the second compression ring may be overall discoid in shape. The second compression ring 124 can further comprise a second gas-pass 147 comprising a first hole 147a, a second hole 147b, a third hole 147c, and a fourth hole 147d, said holes allowing free passage of gases.

As shown in Fig. 5A, the first ring 134 can be attached circumferentially surrounding to the first fluid-pass 126. The second ring 136 can be attached circumferentially surrounding to the second fluid-pass 128. The third ring 138 can be attached to the inner surface 104 along the circumference of the main body 90.

The first strut 140 can extend from the third ring 138 at two points radially opposed in an angle of 180°. The second strut 142 can extend from the third ring 138 to the first ring 134 and is in a 90° angle relative to the first strut 140. The third strut 144 can extend from the third ring 138 to the second ring 136 at a point radially opposed in an angle of 180° to the second strut 142 and may be in a 90° angle relative to the first strut 140. The fourth strut 146 can extend from the first ring 134 to the second ring 136 and cross the first strut 140 in a right angle. Between said struts and rings the holes of the second gas-pass 147 are formed.

The gas-filter membrane 92 is not permeable for microorganisms and is permeable for gases and can be circular, can have a first fenestration 150 and a second fenestration 152, and can be made from PTFE. The gas-filter membrane 92 can be positioned adjacent to the second compression ring 124 such that the second port 130b of the first fluid-pass 126 extends through the first fenestration 150, the second port 132b of the second fluid-pass 128 extends through the second fenestration 152, the inner diameter of the first ring 134 is aligned with the circumference of the first fenestration 150, the inner diameter of the second ring 136 is aligned with the circumference of the second fenestration 152, and the outer diameter of the third ring 138 is aligned with the circumference of the gas-filter membrane 92. The gas-filter membrane 92 can be positioned in between the counter bridge 116 and the first compression ring 94.

The first compression ring 94 can comprise a fourth ring 154, a fifth ring 156, and a sixth ring 158 aligned with the rings 134, 136, 138 of the counter bridge 116, and a fifth strut 160, sixth strut 162, seventh strut 164, and eighth strut 166 aligned with the struts 140, 142, 144, 146 of the counter bridge 116, respectively. Said rings and struts can be in a single plane such that the second compression ring may be overall discoid in shape. The first compression ring 94 can further comprise a first gas pass 167 comprising a fifth hole 167a, a sixth hole 167b, a seventh hole 167c, and an eighth hole 167d, said holes allowing free passage of gases. Said holes of the first gas pass 167 are formed in between the struts and rings of the first compression ring 94. The first compression ring 94 can be positioned adjacent to the gas-filter membrane 92 such that the second port 130b of the first fluid-pass 126 extends through the fourth ring 154, the second port 132b of the second fluid-pass 128 extends through the fifth ring 156, the inner diameter of the fourth ring 154 is aligned with the circumference of the first fenestration 150 of the gas-filter membrane, the inner diameter of the fifth ring 156 is aligned with the circumference of the second fenestration 152 of the gas-filter membrane 92, and the outer diameter of the sixth ring 158 is aligned with the circumference of the gas-filter membrane 92.

The C-clip 96 can be in accordance with DIN 472 for a diameter of 26 mm and can be positioned adjacent to the first compression ring 94 to fix the gas-filter membrane 92 in between the counter bridge 116 and the first compression ring 94.

The alignment of the rings 134, 136, 138 and struts 140, 142, 144, 146 of the counter bridge 116 with the respective rings 154, 156, 158 and struts 160, 162, 164, 166 of the first compression ring 94 and the respective circumference of the gas-filter membrane 92 and its fenestrations 150, 152, advantageously allows a microorganism-tight and secure fixation of the gas-filter membrane 92 in the main body 90, when the C-clip 96 is inserted.

In other preferred embodiments the struts and/or the rings of the counter bridge 116 and the first compression ring 94 can be positioned in another way, if at least the rings are still aligned with the respective circumference of the gas-filter-membrane 92 and fenestrations 150, 152 and if the gas-filter membrane 92 is still microorganism-tight and secure fixated. For example, the first ring 134 and the second ring 136 can be positioned tangentially to the third ring 138 and the fourth ring 154 and fifth ring 156 can be positioned tangentially to the sixth ring 158. In such embodiments, the gas-filter membrane 92 does not comprise a first fenestration 150 and a second fenestration 152, but a first and a second recess (not shown) through which the first and the second fluid-pass extend. In such embodiments, struts can be omitted.

The first tubing hole 120 and the second tubing hole each can be a circular hole in the second connector 110, can have a diameter of 5 mm and may be spanned by the tubing (not shown). The inlet-tube is configured to introduce a fluid to be circulated into the vessel and an outlet-tube is configured to discharge said fluid from said vessel. The first tubing hole 120 can be spanned by the inlet-tube connected to the second port 130b of the first fluid-pass 126. The second tubing hole can be spanned by the outlet-tube connected to the second port 132b of the second fluid pass 128. The first port 132a of the second fluid-pass 128 can be connected to an uptake-tube (not shown) that may extend to the bottom of the vessel.

In other preferred embodiments the tubing hole 120, and/or the second tubing hole can have any shape, for example, oval or polygonal and the diameter can range between 2 mm and 10 mm, depending on the specific tubing used.

The lid 98 can comprise a third connector 168, a circular plate 170, and a cylindrical wall 172.

Fig. 6 shows the gas-exchange unit 200 for allowing gas-exchange of a cell culture medium or a tissue culture medium as a section view in an assembled state.

The circular plate 170 can have a first side 174, a second side 176, and a through-hole 178 comprising a fourth inner thread, into which thread the third connector 168, which can be a pneumatic connector for a 4 mm tubing, can be threaded at the first side 174. The fourth inner thread can be an M5 thread. At the circumference of the circular plate 170, a cylindrical wall 172 may extend at the second side 176, which cylindrical wall 172 can comprise a fifth inner thread 179 configured to be connected to the second connector 110. The fifth inner thread 179 can be an M32x2 thread.

The cylindrical wall 172 can be shaped approximately cylindrical and may comprise a plurality of protrusions and recesses on its outer surface. Those processes and recesses advantageously form a grippy profile for facilitated handling of the gas-exchange unit.

In other preferred embodiments, the cylindrical wall 172 may have another shape and/or another type of grippy profile, as for example a cubic one. In such embodiments, also the circular plate may have another shape, for example, the shape of a square.

In other preferred embodiments the third connector 168 can be any means for connecting two subjects. Examples of connectors are threads, click-connections, plug-connections.

In other preferred embodiments, in which the second connector 110 is no outer thread, the lid 98 may not comprise a fifth inner thread 179 configured to be connected to the second connector 110, but another structure corresponding to the type of the second connector 110.

In other preferred embodiments, the second connector 110 can be configured to be connected directly to the gas-supply. In that case, the second connector-opening 114 preferably has a large diameter allowing sufficient gas trespass when the second connector 110 is not connected to a gas-supply, i.e., when the gas-exchange-unit 200 is placed inside an incubator cabinet. More preferably, in this case the second connector 110 is a threaded connection, a click-connection, or a plug-connection.

The gas-filter membrane 92 can be placed onto the counter bridge 116 such that the first fluid-pass 126 extends through the first fenestration 150 of the gas-filter membrane 92, the second fluid pass 128 extends through the second fenestration 152 of the gas-filter membrane 92, the circumference of the gas-filter membrane 92 and the circumference of the fenestrations 150, 152 of the gas-filter membrane each contact the rings 134, 136, 138, 154, 156, 158 of the counter bridge 116 and the first compression ring 94, in between of which they can be fixed by the pressure exerted by the C-clip 96. The first connector 108 can be screwed onto a threaded 50-mL centrifuge vessel filled with the liquid to be circulated through the fluid-system. The second connector 110 can be screwed into the lid 98 and thus can be connected to the gas-supply intermediary via the lid 98. The third connector can be connected directly to a gas-supply.

When in use, the gas can enter the gas-exchange unit 200 by passing the third connector 168 and subsequently the second connector 110. The gas can then pass the second gas-pass 167. Afterwards, the gas can pass the gas-filter membrane 92 and then the first gas-pass 147 to enter the 50-mL centrifuge vessel. The gas-flow is indicated by arrowheads 169. For the sake of clarity, not each arrowhead 169 is marked with the reference sign 169. In said vessel, the gas may get into contact with the fluid to be circulated, which allows gas-exchange of said fluid.

When connected to a tubing and in use, furthermore, the fluid to be circulated can be introduced into the vessel from the inlet-tube through the first fluid-pass 126 and can be sucked out of the vessel through the uptake-tube and the second fluid-pass 128, into the outlet-tube. The fluid-flow is indicated by arrows 171. For the sake of clarity, not each filled arrow 171 is marked with the reference sign 171.

### 3. System of the invention

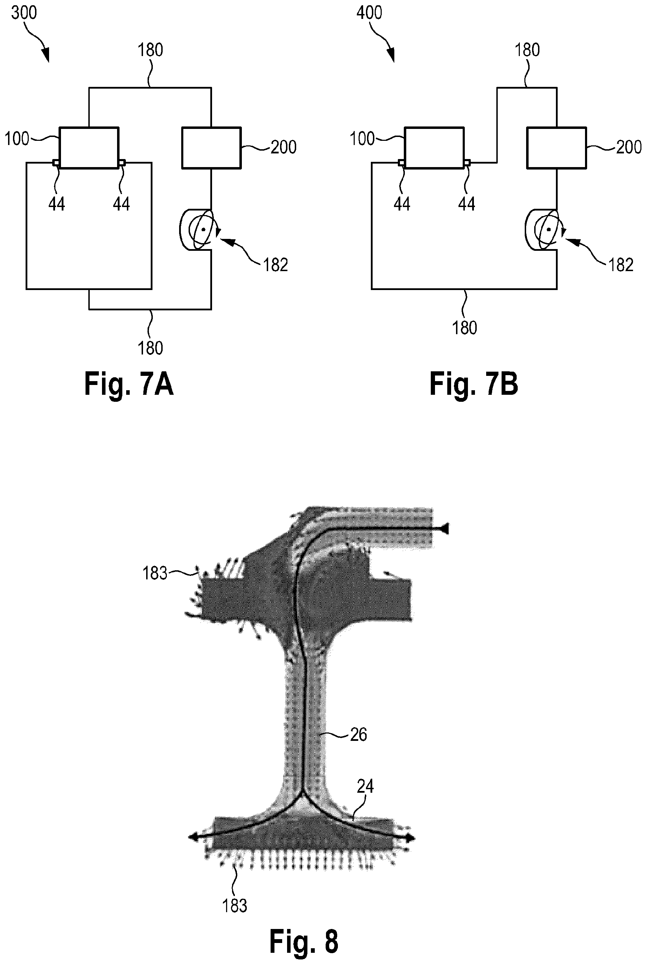

Fig. 7A shows a scheme of a system 300 for culturing a sample 12 comprising tissue or embedded cells. The system 300 comprises a bioreactor 100, a gas-exchange unit 200, and a tubing 180 comprising a pump 182.

When in use, the liquid to be circulated through the fluid-system, usually cell culture medium or tissue culture medium, is circulated by the pump 182. Said liquid may flow from the gas-exchange unit 200 through the tubing 180 into the bioreactor 100, may pass the bioreactor 100, exit the bioreactor 100 into the tubing 180, pass the tubing 180, and enter again the gas-exchange unit 200.

The pump 182 can be a peristaltic pump. In other embodiments, another type of pump can be used, preferably a pump which does not get into direct contact with the fluid to be circulated, i.e., a pump which allows a closed, sterile fluid-system.

When the second section 18 of the bioreactor 100 is opened, the liquid can enter the bioreactor 100 via the inlet comprising a first section 16, a second section 18, and a third section 20 and can exit via the outlets 44, as shown in Fig. 7A.

Fig. 7B shows a system 400, differing from the system 300 in that the second section 18 is closed. In such embodiment, the liquid can enter the bioreactor 100 via an outlet 44 and can exit via another outlet 44.

In other embodiments, the system 300 can comprise a bioreactor not according to the invention or a gas-exchange unit not according to the invention.

In other preferred embodiments, the system 300 or 400 can further comprise an imaging unit (not shown). In such embodiments, the sample bed 22 is at least partially transparent such that the sample 12 can be visually observed. It will be clear to the skilled person that the imaging unit will be positioned close to the transparent sample bed 22.

In other preferred embodiments, the system 300 or 400 can comprise more than one bioreactor 100, and/or more than one gas-exchange unit 200, and/or more than one tubing 180 or pump 182. The system 300 or 400 may also comprise more than one imaging unit. Preferably the system 300 or 400 comprises 2 bioreactors 100 and 2 gas-exchange units 200.

### 4. Production

The bioreactor 100 and the gas-exchange unit 200 can be produced by 3D-printing employing a Rapid Shape D30 II SLA printer. More specifically, the first section 16, the second section 18, and the third section 20 of the bioreactor 100 as well as the main body 90, the compression ring 94, and the lid 98, except the pneumatic connector, of the gas-exchange unit 200 can be 3D-printed. Development and design can be performed in a 3D-computer-assisted-design (3D CAD) software. The raw material for printing can be biocompatible medical resin.

The bioreactor 100 made from biocompatible medical resin proved to be leakage-free and suitable for repeated autoclaving.

Additionally, means configured to be used during assembly and/or autoclaving of the bioreactor 100 and the gas-exchange unit 200 can be 3D printed. Those advantageously allow sterile assembly of the bioreactor 100 and the gas-exchange unit 200.

In a preferred embodiment, the means can be a screwdriver for screwing the second section 18 into the first section 16, a stand for the screwdriver, and/or plugs to seal any ports, for example ports 52 and/or 70, or tubing before autoclaving.

### 5. Simulation of fluid-flow

Fig. 8 shows a simulation of the fluid-flow through a bioreactor 100 comprising a second section 18 opened as to comprise a through-hole 26 in the inverted-funnel-shaped state. Black colour indicates a high speed of the fluid-flow. The arrows indicate the direction of the fluid-flow. In table 1 there are listed the input-parameters used for performing the simulation.

**Table 1: Input-parameters of the simulation of the fluid-flow**

| **Tissue Parameter Value** | | |
|---|---|---|
| 3 mg/ml collagen gel | Permeability | K = 5.0 x 10⁻¹¹ m² |
| 6 mg/ml collagen gel | Permeability | K = 1.5 x 10⁻¹¹ m² |
| Tumor | Permeability | K = 2.5 x 10⁻¹¹ m² |
| RPMI + 10% FCS | Density | 1.007 g/cm³ |
| RPMI + 10% FCS | Dynamic viscosity | 0.958 mPa.s |
| RPMI + 10% FCS | Initial flow speed | 1.5 ml/min |
| RPMI + 10% FCS | Thermal conductivity | 0.60 W/m/°C |
| Skin | Thermal conductivity | 0.37 W/m/°C |

In this embodiment, the fluid-flow can be introduced angled relative to the center axis 88 of the through-hole 26 into the bioreactor 100 approximately in an angle of 90°. When the fluid-flow enters the second hollow cylindrical body 64, exiting the hollow channel 68, the fluid-flow is decelerated due to an enlarged volume inside the second hollow cylindrical body 64 as compared to the hollow channel 68. Then, the fluid-flow can pass the through-hole 26, which may achieve a laminar flow, collimation, and acceleration of the fluid-flow, directs the fluid-flow towards the center of the sample 12, and guides the fluid-flow via the tip of the cone-shaped sample space 24 into the sample 12. The fluid-flow is allowed to exit the sample space 24 evenly towards all directions through the at least one opening 36 circumferentially surrounding the sample space 24, i.e., via the rim of the base of the cone-shaped sample space 24, as indicated by the fluid-flow-vectors 183. Thus, the fluid-flow was focused towards the center of the sample 12.

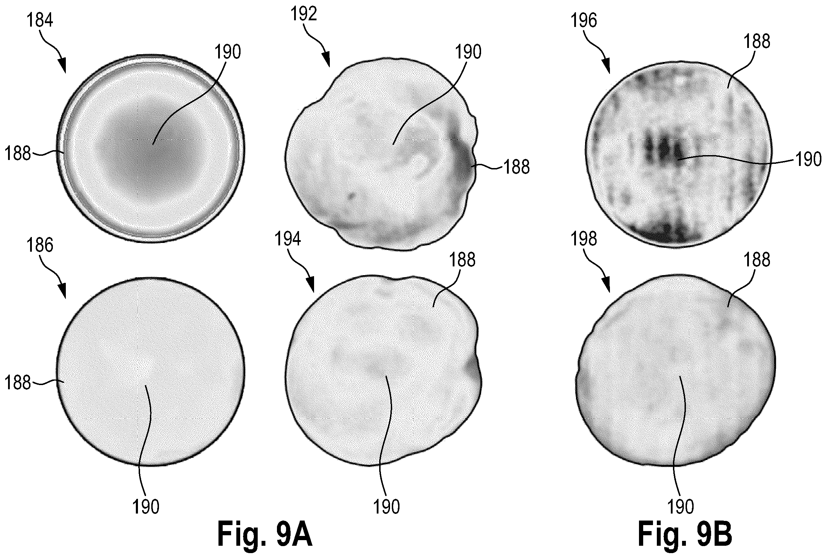

Fig. 9A shows a simulation of the fluid-flow through the sample space 24 when the through-hole 26 is in the cylindrical-shaped state (top panel, 184) or in the inverted-funnel-shaped state (bottom panel, 186) as a top view onto the sample 12. Black colour indicates a higher perfusion. The sample 12 has a rim 188 and a center 190.

When the through-hole 26 is in the cylindrical-shaped state (top), the fluid-flow is highest and thus the perfusion the most intensive at the rim 188 of the sample 12, close to the at least one opening 36. In the center 190 of the sample 12, the fluid-flow is low.

When the through-hole 26 is in the inverted-funnel-shaped state (bottom), the fluid-flow is overall more homogeneous and especially enhanced in the center 190 of the sample 12. At the rim 188 of the sample 12, the fluid-flow is reduced compared to when the through-hole 26 is in the cylindrical-shaped state, but still not low.

### 6. Cell culture

Cell-culture was performed on a bioreactor 100. The sample 12 was cells expressing nuclear green fluorescent protein (GFP) that were embedded in a collagen-hydrogel having a diameter of 6 mm and a thickness of 2 mm. The sample 12 was inserted into the sample bed 22 and the bioreactor 100 was assembled. Afterwards, the system 300 was assembled from the bioreactor 100, the gas-exchange unit 200, and a tubing 180 comprising a peristaltic pump. The sample 12 was cultured for 5 min in the system 300 at a perfusion rate of 1.5 mL/min with cell culture medium supplemented with the nuclear dye DRAQ5 (1,5-Bis{[2-(Di-methylamin)-Ethyl]amin}-4, 8-Dihydroxyanthracen-9,10-dion). The second section 18 comprised a through-hole 26 in either the inverted-funnel-shaped state or a cylindrical shaped state. As a control, identical samples 12 were cultured for 5 min (negative control) or 12 h (positive control) in a cell culture dish with cell culture medium supplemented with DRAQ5. All samples were washed, fixed, optically cleared and 3D imaged on an Ultramicroscope II light sheet system covering the whole sample within the field of view. The signals from GFP and DRAQ5 were detected within individual channels. To avoid an effect of chromatic aberration mismatch and other optical artifacts, fluorescence intensities of positive nuclei were measured for each channel on the center voxel of their intensity maxima identified with a "Difference of Gaussian" (DoG) algorithm maxima and convolved with a 2D gaussian filter. To exclude light absorption and scattering effects, the perfusion-dependent signal from DRAQ5 was normalized to the perfusion-independent GFP signal and the resulting image stacks were summed along the z-axis.

In other embodiments, the sample can have, for example, a thickness of 1 mm, 2 mm, 3 mm, 4 mm or 5 mm, preferably the sample has a thickness of 2 mm.

Fig. 9B shows a visualization of the concentration of DRAQ5 as a measure of the fluid-flow through the sample 12 (top left circle 192 and bottom left circle 194) cultured in the system 300 as a top view onto the sample 12. Furthermore, the negative control (top right circle 196) and a positive control (bottom right circle 198) are shown for comparison. Black colour indicates a higher DRAQ5-concentration. In the negative control, the perfusion is very low overall at the rim 188 and the center 190, while in the positive control, the sample space is evenly high stained by DRAQ5 at the rim 188 and the center 190. The experiment confirms the data from the simulation shown in Fig. 9A. While the through-hole 26 in the cylindrical-shaped state (left top circle) sustains perfusion especially at the rim 188, the through-hole 26 in the inverted-funnel-shaped state (left bottom circle) sustains a more homogeneous perfusion and more extensive and perfusion especially in the center 190, but also to a sufficient degree at the rim 188.

### 7. Tissue culture

Tissue-culture was performed on a system 300. The sample 12 is a living murine liver biopsy having a diameter of 6 mm and a thickness of 2 mm. The sample 12 was inserted into the sample bed 22 and the bioreactor 100 was assembled. The sample 12 was immobilized by the mesh 85 and the compression by the second section 18. The second section 18 was opened as to comprise a through-hole 26. The through-hole 26 had the inverted-funnel-shaped state. Thus, the sample 12 was immobilized at the base of the cone-shape sample space 24. Afterwards, the system 300 was assembled from the bioreactor 100, the gas-exchange unit 200, and a tubing 180 comprising a pump 182 being a peristaltic pump. The fluid-flow was focused towards the center of the sample 12 by guiding the fluid-flow via the tip of the cone-shaped sample space 24 into the sample 12 and allowing the fluid-flow to exit the sample 12 via the rim of the base of the cone-shaped sample space 24, as shown in Fig. 8. and the sample 12 was cultured for 2 days in the system 300 at a perfusion rate of 1.5 mL/min with tissue culture medium. As a control, identical samples 12 were cultured for 2 days in a cell culture dish (static control) with tissue culture medium or immediately fixed at the onset of culturing (d0 culture). All samples 12 were fixed and embedded in paraffin. Serial sections were cut.

In other embodiments, the sample can have, for example, a thickness of 1 mm, 2 mm, 3 mm, 4 mm or 5 mm, preferably the sample has a thickness of 2 mm.

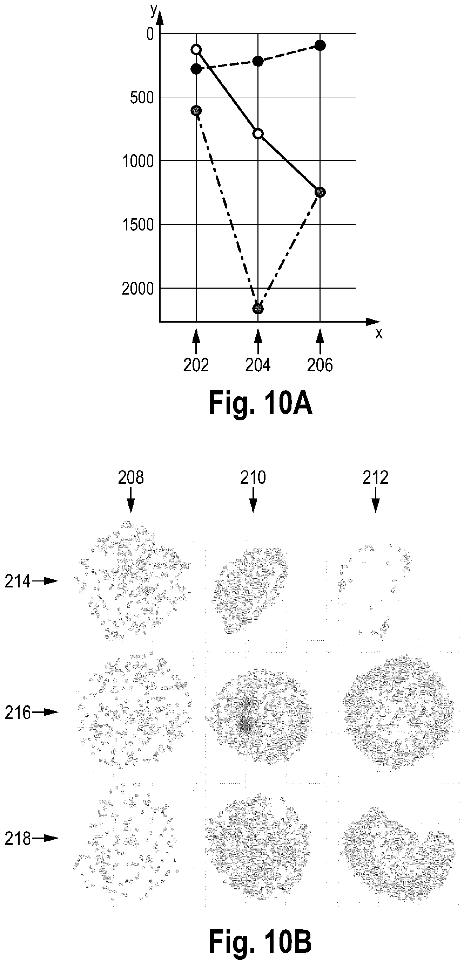

From all samples 12 it was chosen each one section originating from the same height of the biopsy's top section facing the outflow-end of the second section 18, the middle section, and the bottom section (facing the cover slip). The sections were immunofluorescent stained for cleaved caspase-3, which is an apoptosis (cell death) marker. The sections were imaged with an epifluorescence microscope covering the whole tissue area in a mosaic scan. For all sections, cleaved caspase-3 positive cells were segmented and separated with the same threshold. The total number of cleaved caspase-3 positive cells was normalized to the total area of the section (Fig. 10A) or locally summarized within an array of 40 × 40 bins to visualize apoptotic hotspots (Fig. 10B). The more cleaved caspase-3 positive cells are present, the less is the viability of the sample.

Fig. 10A shows that the viability of the sample of the dO-control (dashed line) is more or less equal in all tissue sections. In the top tissue section (panel 202 on the x-axis), the bioreactor 100 (full line) slightly enhances the viability over the dO-control. Static culture (dot-dashed line) resulted in a slight reduction of viability compared to the dO-control. In the middle tissue section (panel 204 on the x-axis), static culture heavily reduced viability, while culture in the bioreactor 100 lead to an only slight reduction of viability. In the bottom tissue section (panel 206 on the x-axis), the viability of the sample in static culture and cultured in bioreactor 100 is equally and moderately reduced.

Fig. 10B shows that the viability of the sample of the dO-control (column 208) is more or less equal in all tissue sections. In the top tissue section (row 214), the bioreactor 100 (column 212) enhances the viability over the dO-control and the static control (column 210). In the middle tissue section (row 216), static culture heavily reduced viability. Especially in the center 190 there is a spot of cleaved caspase-3 positive (i.e., dying) cells. Culture in the bioreactor 100 lead to a reduction and homogeneous distribution of spots of cleaved caspase-3 positive cells. Especially in the center 190, only few cleaved caspase-3 positive cells are detected. In the bottom tissue section (row 218), the viability of the sample in static culture and cultured in bioreactor 100 is equally and moderately reduced.

Thus, especially in the middle section, i.e., in the center 190 of the sample 12 (i.e., in the center 190), the viability of the sample 12 can be enhanced by the use of a bioreactor according to the invention.

### 8. Live cell imaging

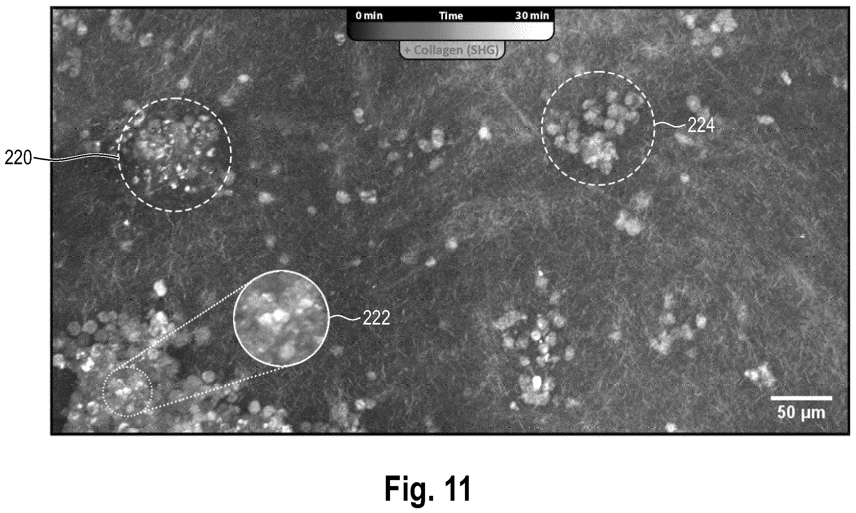

Fig. 11 shows a time projection from a 30-minute video acquired during culture of an ovarian biopsy for 4 days in a system 300.

A living biopsy of a mouse ovary that contained fluorescent mCherry tagged metastatic breast cancer cells (MMPV-PyMT-ML1B1B1) was inserted into the sample bed 22 and the bioreactor 100 was assembled. The sample 12 was immobilized by the mesh 85 and the compression by the second section 18. The second section 18 was opened as to comprise a through-hole 26. The through-hole 26 had the inverted-funnel-shaped state. Thus, the sample 12 was immobilized at the base of the cone-shape sample space 24. No lid and no gas-supply were connected to the second connector 110 of the gas-exchange unit 200. The gas-exchange unit 200 was connected via the first connector 108 to a 50-mL centrifuge vessel containing a tissue culture medium. The system 300 except the pump 182 and a part of the tubing 180 was placed into an incubator cabinet and the pump was activated. The fluid-flow was focused towards the center of the sample 12 by guiding the fluid-flow via the tip of the cone-shaped sample space 24 into the sample 12 and allowing the fluid-flow to exit the sample 12 via the rim of the base of the cone-shaped sample space 24, as shown in Fig. 8 and the sample 12 was cultured for four days in the system 300 at a perfusion rate of 1.5 mL/min. Afterwards, the bioreactor 100 was transferred into a microscope stage top incubator (PECON) located on top of an inverted multi-photon microscope and the gas-exchange unit 200 was transferred together with a rack holding it into a heatable water bath assembled from a water-filled beaker placed into a bottle heater socket (PECON). It is understood that the tubing and the pump were arranged accordingly. The lid was connected to the second connector 110. The CO₂ supply was connected to the gas-exchange unit 200 via the third connector 168 being a pneumatic connector. Culturing was continued.

Imaging was performed outside the incubator cabinet with an inverted multi-photon microscope. Connective tissue (collagen) was detected by the second harmonics generation (SHG) signal. The breast cancer metastasis was detected by the fluorescent signal from mCherry. The brighter the detected signal from mCherry is shown in Fig. 11, the later during the 30-minutes video it exists. In Fig. 11, circle 220 highlights a group of cells that undergo strong shrinkage during the 30-minutes video. Circle 222 highlights movement of cells. Circle 224 highlights stationary cells.

Thus, it is possible to observe the sample 12 visually during tissue culture or cell culture in the system 300, i.e., in the bioreactor 100. Moreover, it is possible to perform ex vivo live cell imaging during tissue culture or cell culture. Furthermore, the sample 12 can be imaged to generate pictures and/or videos.

### 9. Conclusion

The inventors provide a highly flexible system 300 or 400 from a bioreactor 100 and a gas-exchange unit 200 allowing cell culture or tissue culture of thick as well as thin samples 12 in an over-flow as well as a perfusion manner, said system 300 or 400 further allowing for ex vivo live cell imaging.

## Claims

1. A bioreactor (100) for culturing a sample (12) comprising tissue or embedded cells, said bioreactor (100) comprising an inlet configured to introduce a fluid into the bioreactor (100), said inlet comprising
- a first section (16) comprising a sample bed (22), said sample bed (22) being configured to hold the sample (12) in a sample space (24), in which the sample (12) is located, when the sample (12) was inserted,
- a second section (18) being movable relative to the sample bed (22) to compress the sample (12) in between the second section (18) and the sample bed (22), when the sample (12) has been inserted, and being openable as to comprise a through-hole (26) having a diameter x, said through-hole (26) being open towards the center of the sample space (24) and configured for guiding a fluid-flow onto the center of the sample space (24), and
- a third section (20) configured to be connected to a fluid-supply.

2. The bioreactor (100) of claim 1, wherein the through-hole (26) is adjustable between
an inverted-funnel-shaped state, such that the through-hole (26) comprises a narrowest section (29) and a conical section (30) and such that the sample space (24) is cone-shaped wherein the conical section (30) forms the tip (32) of the cone-shaped sample space (24), and
a cylindrical-shaped state (34), wherein the diameter x of the through-hole (26) in the cylindrical-shaped state (34) is wider than the diameter x at the narrowest section (29) of the through-hole (26) in the inverted-funnel-shaped state.

3. The bioreactor (100) of any one of the preceding claims, wherein at least one opening (36) is formed between the second section (18) and the sample bed (22), said opening being arranged circumferentially surrounding the sample space (24).

4. The bioreactor (100) of claim 3, wherein the at least one opening (36) are two separated sickle-shaped openings (86).

5. The bioreactor (100) of any one of the preceding claims, wherein the first section (16) comprises a first hollow cylindrical body (40) comprising a first inner thread (58) located on the inner surface of the first hollow cylindrical body (40), and
wherein the second section (18) comprises a first outer thread (76) that can be screwed into the first inner thread (58), wherein the second section (18) is movable by screwing it in or out of the first inner thread (58).

6. The bioreactor (100) of claim 5, wherein the first hollow cylindrical body (40) comprises a second outer thread (60) located on the outer surface of the first hollow cylindrical body (40), and the third section (20) comprises a second inner thread (66) that can be screwed onto the second outer thread (60).

7. The bioreactor (100) of any one of the preceding claims, wherein the sample bed (22) is at least partially transparent such that the sample (12) can be visually observed.

8. The bioreactor (100) of any one of the preceding claims comprising a mesh (85), wherein the mesh (85) is located in between the sample bed (22) and the second section (18) and wherein the sample (12), when inserted, is enclosed between the mesh (85) and the sample bed (22).

9. A gas-exchange unit (200) for allowing gas-exchange of a cell culture medium or a tissue culture medium during cell culture or tissue culture, the gas-exchange unit (200) comprising
- a main body (90) comprising
a tubular lumen,
a first connector (108), configured to be connected to a vessel, and allowing free passage of gases,
a second connector (110), configured to be at least intermediary connected to a gas-supply, and allowing free passage of gases, and
- a gas-filter membrane (92),
wherein the gas-filter membrane is not permeable for microorganisms, is permeable for gases, and spans the tubular lumen between the first connector (108) and the second connector (110), such that a gas passing the tubular lumen from the second connector (110) to the first connector (108) passes the gas-filter membrane (92).

10. The gas-exchange unit (200) of claim 9, wherein the gas-exchange unit (200) comprises an inlet-tube configured to introduce the fluid to be circulated into the vessel the first connector (108) is configured to be connected to, an outlet-tube configured to discharge said fluid from said vessel, and preferably a counter bridge (116) comprising a first fluid-pass (126) and a second fluid-pass (128), each fluid-pass extending through a plane of the gas-filter membrane, and an uptake-tube extending from the second fluid-pass (128) to the bottom of the vessel the first connector (108) is configured to be connected to, wherein the inlet-tube is connected to the first fluid-pass (126) and extends inside the second connector (110) and the outlet-tube is connected to the second fluid-pass (128) and extends inside the second connector (110), and wherein the inlet-tube and the outlet-tube preferably extend each via a tubing hole (120) in the second connector (110) and wherein the first-fluid pass (126) and the second fluid-pass (128) preferably extend each via a fenestration (150, 152) in the gas-filter membrane.

11. The gas-exchange unit (200) of any one of claims 9 to 10, comprising a lid (98), said lid (98) comprising a third connector (168) to be connected directly to a gas-supply, and wherein the first connector (108), the second connector (110), and the third connector (168) each preferably comprise a thread such that the first connector (108) is configured to be screwed onto a threaded vessel, and the second connector (110) is configured to be connected to the gas-supply via a screw connection to the lid (98), and wherein the third connector (168) is preferably a pneumatic connector.

12. A system (300; 400) for culturing a sample (12) comprising tissue or embedded cells, the system (300;400) comprising
- at least one bioreactor (100) of any one of claims 1 to 8,
- at least one gas-exchange unit (200) of any one of claims 9 to 11, and
- a tubing (180) comprising a pump (182).

13. The system of claim (12), wherein the system (300; 400) further comprises an imaging unit configured for imaging the sample (12).

14. A method for culturing a sample (12) having a thickness of more than 1 mm comprising tissue or embedded cells, said method comprising the following steps:
- immobilizing the sample (12) at a base of a cone-shape sample space (24),
- focusing a fluid-flow towards the center of the sample (12) by guiding the fluid-flow via the tip of the cone-shaped sample space (24) into the sample (12) and allowing the fluid-flow to exit the sample (12) via a rim of the base of the cone-shaped sample space (24), and
- culturing the sample (12) for a desired period.

15. The method of claim 14, wherein the method further comprises the use of a bioreactor (100) of any one of claims 1 to 8 or a system (300; 400) of any one of claims 12 to 13, and preferably further comprises the step of imaging the sample (12) when the system (300; 400) comprises the imaging unit.
